# FASCICULE DE BREVET EUROPEEN

(11) **EP 0 898 958 B1**
(45) Date de publication et mention de la délivrance du brevet: **27.06.2001**
(21) Numéro de dépôt: 98401642.8
(22) Date de dépôt: 01.07.1998
(51) Int. Cl.: A61K 7/48

(54) **Composition filmogène épaissie**
Verdickte filmbildende Zusammensetzung
Thickened film-forming composition

(30) Priorité: 28.08.1997 FR 9710759
(43) Date de publication de la demande: 03.03.1999
(73) Titulaire: L'OREAL, 75008 Paris (FR)
(72) Inventeur: Ramin, Roland, 75014 Paris (FR)
(74) Mandataire: Lhoste, Catherine

(56) Documents cités:
- EP-A- 0 455 073
- WO-A-94/18935
- WO-A-96/02225
- WO-A-96/23482
- DE-A- 19 522 750

## Description

L'invention a pour objet une composition filmogène comprenant un polymère filmogène et un nouvel épaississant utilisable dans le domaine cosmétique. L'invention se rapporte aussi à une utilisation de cette composition pour le traitement et le soin des matières kératiniques telles que la peau, les ongles, les cils, les sourcils, les cheveux ou des muqueuses telles que les lèvres et l'intérieur des paupières. Elle est destinée plus spécialement au traitement et au soin des ongles.

De façon plus précise, l'invention se rapporte à une composition contenant des polymères filmogènes, capable de former sur un support (ongle, cil, cheveu) un film homogène et continu.

Dans les compositions filmogènes comme les vernis à ongles, il est courant d'épaissir la phase organique par des agents épaississants. Les compositions épaissies permettent de faciliter la prise du produit hors de son conditionnement sans perte significative, de répartir le produit de façon régulière sur la zone à traiter ou bien encore de pouvoir utiliser le produit dans des quantités suffisantes pour obtenir l'effet cosmétique recherché. De plus, pour des compositions comprenant une quantité de pigments comme les vernis à ongles, l'agent épaississant permet d'empêcher la sédimentation des pigments lors du stockage.

Pour épaissir les compositions, il est connu d'utiliser des argiles telles que les montmorrillonites organomodifiées comme décrit dans la demande GB-A-2021411. Mais la préparation de telles compositions nécessite de bien disperser l'argile dans la composition. Ainsi, la dispersion doit nécessairement s'effectuer à l'aide d'un homogénéisateur haute pression Gaulin ce qui conduit à une étape contraignante, longue et coûteuse dans la fabrication des compositions. Il est donc souhaitable de disposer d'un épaississant qui soit facile à mettre en oeuvre. En outre, les argiles comme les montmorillonites organomodifiées modifient les propriétés du film obtenu après l'application de la composition. En effet, on constate que le film est moins résistant aux chocs et s'écaille plus facilement.

Les silices hydrophiles et hydrophobes sont également connues comme agents épaississants dans les vernis à ongles, notamment dans la demande FR-A-1453089. Si les silices ne nuisent pas aux propriétés du film obtenu après application de la composition, elles sont toutefois difficiles à mettre en oeuvre, nécessitant aussi une étape de mise en dispersion. De plus, les silices ont tendance à matifier la composition filmogène et le film obtenu, conduisant à la formation de films mats ou satinés. Leur emploi n'est donc pas préconisé pour la préparation de compositions filmogènes transparentes et pour l'obtention de film brillant.

Le but de la présente invention est de proposer une composition filmogène transparente épaissie, bien adaptée au soin des ongles, présentant de bonnes propriétés cosmétiques et ne présentant pas les inconvénients mentionnés ci-dessus.

La Demanderesse a découvert qu'une telle composition pouvait être obtenue en utilisant un épaississant particulier.

La présente invention a donc pour objet une composition comprenant un polymère filmogène et une phase organique, caractérisée par le fait qu'elle comprend un alkyléther de polysaccharide formé de motifs comportant au moins deux cycles osidiques différents, chaque motif comportant au moins un groupe hydroxyle substitué par une chaîne alkyle hydrocarbonée saturée, la phase organique comprenant au moins un milieu solvant de l'alkyléther de polysaccharide.

Grâce à l'alkyléther de polysaccharide, la composition selon l'invention est transparente et présente une viscosité satisfaisante permettant un bon étalement de la composition. Cet épaississant convient parfaitement à la préparation de compositions pigmentées stables dans le temps, comme les vernis à ongles.

Dans l'épaississant de l'invention, on entend par « chaîne alkyle hydrocarbonée » une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment méthyle, éthyle,n-propyle, isopropyle, n-butyle, isobutyle, tertiobutyle, n-pentyle. Ces alkyléthers peuvent être fabriqués comme décrits dans les documents EP-A-281 360, EP-A-708 114, EP-A-281360.

Selon un mode de réalisation préféré de l'invention, l'alkyléther de polysaccharide a un poids moléculaire moyen en poids supérieur à 100 000, et de préférence supérieur à 200 000. Ce poids moléculaire peut aller jusqu'à 1 million. Cet alkyléther peut comporter de un à six et mieux de deux à quatre groupes hydroxyle par motif, substitués par une chaîne alkyle hydrocarbonée saturée ou non.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique. Comme gommes appropriées, on peut citer par exemple la gomme de guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme de guar. Ainsi, avantageusement l'alkyéther est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,8, tel que décrit dans les documents RD 95378007 (octobre 1995) et EP-A-708114. Cette gomme est en particulier celle vendue par la société Aqualon sous les noms N-HANCE-AG 200® et N-HANCE AG 50® .

La concentration en alkyléther dépend de la forme galénique, de la consistance recherchées pour la composition ainsi que de la quantité de phase organique à épaissir. En particulier le rapport en poids de la quantité de phase grasse liquide sur la quantité d'épaississant est choisi par exemple dans la gamme allant de 5 à 500. La composition selon l'invention peut contenir par exemple une quantité d'alkyléther de polysaccharide allant de 0,2 à 20 % du poids total de la composition, et de préférence de 1,5 à 8 %.

Selon l'invention, le milieu solvant de l'alkyléther de polysaccharide présent dans la composition peut être un solvant organique ou une huile. En d'autres termes, l'alkyléther de polysaccharide est un épaississant des solvants organiques et des huiles. Par huiles, on entend toute matière grasse liquide à température ambiante.

Le solvant organique peut être, par exemple, choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde;

Ces solvants conviennent plus particulièrement pour le maquillage et le soin des ongles : la composition constitue alors un vernis à ongles ou un produit de soin des ongles.

Parmi les huiles utilisables comme milieu solvant de l'alkyléther de polysaccharide selon l'invention, on peut citer par exemple :
- les huiles d'origine végétale comme les triglycérides liquides, par exemple les huiles de tournesol, de maïs, de soja, de jojoba, de courge, de pépins de raisin, de sésame, de noisette, d'abricot, de macadamia, de ricin, les triglycérides des acides caprylique/caprique comme ceux vendus par la société STEARINERIES DUBOIS ou ceux vendus sous les dénominations MIGLYOL 810, 812 et 818 par la société DYNAMIT NOBEL.
- les huiles d'origine animale telle que la lanoline,
- les huiles d'origine minérale,
- les huiles de synthèse comme les alcools gras tels que octyl-2-dodécanol; les esters et en particulier les esters d'acides gras, et notamment les esters ayant un nombre total d'atomes de carbone choisi entre 12 et 80 et mieux entre 16 et 50; les silicones phénylées, et notamment les phényl triméthicones, les diphényl diméthicones, les polyméthylphényl siloxanes.

L'homme du métier sait, par ses connaissances, déterminer par de simples essais de routine les huiles solubilisant l'alkyléther de polysaccharide.

Ces huiles solvant de l'alkyléther de polysaccharide conviennent plus particulièrement pour la préparation de produit de soin des ongles.

Des huiles complémentaires, non solvant de l'alkylléther de polysaccharide, peuvent en outre être ajoutées dans la composition. Comme huile complémentaire, on peut notamment citer les résines et les gommes liquides à température ambiante de silicone, les huiles hydrocarbonées partiellement fluorées, les huiles perfluorées, les huiles siliconées exemptes de groupements aromatiques telles que les polysiloxanes linéaires ou ramifiés comme les polydiméthylpolysiloxanes, les polyéthylméthylpolysiloxanes, polyalkylméthylsiloxanes et les polysiloxanes cycliques tels que octaméthylcyclotétrasiloxane, décaméthylcyclopentasiloxane ou leurs mélanges ; les huiles de silicones fluorées ; les polysiloxanes fonctionnalisés par une ou plusieurs fonctions hydroxyles et/ou un ou plusieurs groupements polyéthers tels que les diméthicones copolyols ; les hydrocarbures linéaires ou ramifiés, comme l'huile de vaseline, l'isohexadécane, l'isododécane.

Les solvants de l'alkyléther de polysaccharide (solvant organique ou huile) peuvent être présents à raison de 40 à 99,3 % en poids, par rapport au poids total de la phase organique de la composition, et mieux de 72 % à 98,5 %. Les huiles complémentaires peuvent être ajoutées dans la composition en une quantité pouvant aller de 0 % à 75 % en poids, par rapport au poids total de la phase organique, et mieux de 0 % à 50 % en poids.

Le polymère filmogène présent dans la composition selon l'invention peut être tout polymère couramment utilisé dans les vernis à ongles en milieu solvant, bien connu de l'homme du métier. A titre d'exemple, le polymère peut être choisi parmi la nitrocellulose, l'acétobutyrate de cellulose, les butyralpolyvinyliques, les résines alkydes, les résines résultant de la condensation de formaldéhyde avec une arylsulfonamide, les polyesters, les polyuréthannes, les polyester-polyuréthanes, les polyéther-polyuréthanes, les polymères radicalaires, notamment de type acrylique, acrylique styrène et/ou vinylique et leurs mélanges.

Les polymères peuvent être dissous ou dispersés dans la composition. Ils peuvent être généralement présents à une teneur allant de 0,5 % à 40 % en poids par rapport au poids total de la composition, et mieux allant de 10 % à 20 % en poids.

Grâce à la présence de l'alkyléther de polysaccharide, il est possible d'utiliser, en association, de la silice pyrogénée, notamment pour ajuster la viscosité de la composition, sans nuire à la brillance du film.

La silice pyrogénée peut se présenter sous forme de silice pyrogénée hydrophile ou de silice pyrogénée hydrophobe.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130® ", "AEROSIL 200® ", "AEROSIL 255® ", "AEROSIL 300® ", "AEROSIL 380® " par la société Degussa, "CAB-O-SIL HS-5® ", "CAB-O-SIL EH-5® ", "CAB-O-SIL LM-130® ", "CAB-O-SIL MS-55® ", "CAB-O-SIL M-5® " par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812® " par la société Degussa, "CAB-O-SIL TS-530® " par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972® ", "AEROSIL R974® " par la société Degussa, "CAB-O-SIL TS-610® ", "CAB-O-SIL TS-720® " par la société Cabot.

La silice pyrogénée présente de préférence une taille de particules pouvant être nanométrique à micrométrique, par exemple allant d'environ de 5 à 200 nm.

La silice pyrogénée peut être présente dans la composition selon l'invention en une quantité allant de 0,1 % à 5 % en poids, par rapport au poids total de la phase organique de la composition, de préférence de 0,5 % à 1 % en poids.

Il est également possible d'introduire dans la composition une argile comme les bentonites organomodifiées, sans nuire à la propriété du film, grâce à la présence de l'alkyléther de polysaccharide. Cette argile peut être présente en une quantité allant de 0,1 % à 3 % en poids, par rapport au poids total de la composition, et mieux de 0,5 % à 1,5 % en poids. Comme bentonites, on peut utiliser celles vendues sous les dénominations "Bentone 27® ", "Bentone 34® ", "Bentone 38® " par la société Rheox, ou encore sous la dénomination "Tixogel LG® " par la société Sud Chemie.

La composition selon l'invention peut également comprendre, en plus du polymère filmogène, des agents plastifiants qui permettent de régler la flexibilité du film sans affaiblir sa résistance physique.

Les agents plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle; les citrates de triéthyle, de tributyle, l'acétyl-citrate de tributyle; les phosphates de tributyle, de triphényle; les glycols; le camphre ainsi que leurs dérivés et leurs mélanges. Les plastifiants peuvent être généralement présents à une teneur allant de 1 % à 30 % en poids par rapport au poids total de la composition, et mieux de 5 % à 10 % en poids.

Par ailleurs, la composition selon l'invention peut contenir des adjuvants couramment utilisés dans les compositions cosmétiques. On peut citer à titre d'exemple d'adjuvants les colorants, les pigments, les nacres, les laques, les agents anti-UV, les agents épaississants, les tensioactifs, les cires, les parfums, et des actifs de tels que le D-panthénol, le phytantriol, les vitamines et leurs dérivés, la kératine et ses dérivés, la mélanine, le collagène, la cystine, le chitosane et ses dérivés, la biotine, les oligo-éléments, la glycérine, les hydrolysats de protéines, les phospholipides, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être avantageusement utilisée pour le traitement, le maquillage, ou le soin des matières kératiniques et/ou des muqueuses selon la nature des actifs utilisés. La composition de maquillage peut être un vernis à ongles, un eye-liner, un mascara, un fond de teint, un anti-cernes, un fard à paupières ou à joues, ou bien encore un rouge à lèvres.

La composition selon l'invention peut avantageusement se présenter sous forme d'un vernis à ongles ou d'une composition de soin des ongles. Aussi, l'invention a encore pour objet un composition de soin ou de vernis à ongles comprenant un polymère filmogène, une phase organique et un alkyléther de polysaccharide tels que définis précédemment.

L'invention se rapporte également à l'utilisation d'un alkyléther de polysaccharide tels que défini précédemment comme agent épaississant d'une composition contenant un polymère filmogène et une phase organique comprenant au moins un milieu solvant dudit alkyléther.

L'invention se rapporte aussi à un procédé de traitement cosmétique ou de maquillage des matières kératiniques, et notamment des ongles, et/ou des muqueuses, consistant à appliquer sur les matières kératiniques et/ou les muqueuses une composition telle que décrite précédemment.

On va maintenant donner des exemples illustrant la présente invention sans toutefois la limiter.

### Exemple 1 :

On a préparé une composition de vernis à ongles ayant la composition suivante :
- polymères filmogènes (nitrocellulose, résine) 28 g
- plastifiant 7 g
- alcool isopropylique 5 g
- guar éthylé de degré de substitution d'environ 2,5 (1) 3 g
- pigments 1 g
- acétate de éthyle/acétate de butyle qsp 100 g
   (1) vendu sous la dénomination N-HANCE AG 200® par Aqualon

Après application de la composition sur l'ongle et après séchage, on obtient un film lisse, homogène et brillant.

### Exemple 2 :

On a préparé un produit de soin des ongles ayant la composition suivante :
- polymères filmogènes (nitrocellulose, résine) 14 g
- plastifiant 3 g
- filtre UV 0,5 g
- colorants 0,1 g
- silice pyrogénée (Degussa 200) 0,5 g
- isopropanol 5 g
- guar éthylé de degré de substitution d'environ 2,5(1) 0,5 g
- D-Panthénol 0,5 g
- phytantriol 0,1 g
- acétate de butyle/acétate d'éthyle qsp 100 g
(1) vendu sous la dénomination N-HANCE AG 200® par Aqualon

La composition s'applique facilement sur l'ongle et laisse après séchage un film homogène et brillant qui embellit l'état des ongles.

### Exemple 3 :

On a préparé une huile de soin pour ongles ayant la composition suivante :
- acétobutyrate de cellulose 0,5 g
- huile minérale 5 g
- silice pyrogénée (Degussa 200) 0,5 g
- guar éthylé de degré de substitution d'environ 2,5(1) 0,5 g
- additifs (actifs et colorants) 1 g
- alcool isopropylique 5 g
- monométhyl éther de propylène glycol 3 g
- huile de silicone volatile 20 g
- huile végétale qsp 100 g
(1) vendu sous la dénomination N-HANCE AG 200® par Aqualon

Cette huile de soin s'applique facilement sur l'ongle et les cuticules ; elle pénètre par massage dans la matrice de l'ongle et dans la tablette unguéale.

## Revendications

1. Composition comprenant un polymère filmogène et une phase organique, caractérisé par le fait qu'elle comprend un alkyléther de polysaccharide formé de motifs comportant au moins deux cycles osidiques différents, chaque motif comportant au moins un groupe hydroxyle substitué par une chaîne alkyle hydrocarbonée saturée, la phase organique comprenant au moins un milieu solvant de l'alkyléther de polysaccharide.

2. Composition selon la revendication 1, caractérisée par le fait que deux à quatre groupes hydroxyle par motif sont substitués par une chaîne alkyle hydrocarbonée saturée.

3. Composition selon l'une quelconque des revendications précédentes, caractérisée par le fait que la chaîne alkyle hydrocarbonée saturée comporte de 1 à 24 atomes de carbone.

4. Composition selon l'une quelconque des revendications précédentes, caractérisée par le fait que la chaîne alkyle hydrocarbonée saturée comporte de 2 à 10 atomes de carbone.

5. Composition selon l'une quelconque des revendications précédentes, caractérisée par le fait que la chaîne alkyle est choisie dans le groupe formé par les radicaux méthyle, éthyle, n-propyle, isopropyle, n-butyle, isobutyle, tertiobutyle.

6. Composition selon l'une quelconque des revendications précédentes, caractérisée par le fait que les cycles osidiques sont choisis dans le groupe formé par le mannose, le galactose, le glucose, le furanose, le rhamnose, l'arabinose.

7. Composition selon l'une quelconque des revendications précédentes, caractérisée par le fait que l'alkyléther de polysaccharide est un alkyléther d'une gomme choisie parmi la gomme de guar, la gomme de caroube, la gomme de karaya, la gomme adragante et leurs mélanges.

8. Composition selon l'une quelconque des revendications précédentes, caractérisée par le fait que l'alkyéther est un galactomannane alkylé de chaîne alkyle en C₁ à C₆ et mieux en C₁ à C₃.

9. Composition selon l'une quelconque des revendications précédentes, caractérisée par le fait que l'alkyléther de polysaccharide est de la gomme de guar à chaîne éthyle avec un degré de substitution de 2 à 3.

10. Composition selon l'une quelconque des revendications précédentes, caractérisée par le fait que l'alkyléther de polysaccharide a un poids moléculaire moyen en poids supérieur à 200 000.

11. Composition selon l'une quelconque des revendications précédentes, caractérisée par le fait que l'alkyléther de polysaccharide est présent en une quantité telle que le rapport (en poids) de la quantité de phase grasse liquide sur la quantité dudit alkyléther est choisi dans la gamme allant de 5 à 500.

12. Composition selon l'une quelconque des revendications précédentes, caractérisée par le fait que l'alkyléther de polysaccharide est présent en une quantité allant de 0,2 à 20 % du poids total de la composition et mieux de 1,5 à 8 % du poids total de la composition.

13. Composition selon l'une quelconque des revendications précédentes, caractérisée par le fait que le polymère filmogène est choisi dans le groupe formé par la nitrocellulose, l'acétobutyrate de cellulose, les butyralpolyvinyliques, les résines résultant de la condensation de formaldéhyde avec une arylsulfonamide, les résines alkydes, les polyesters, les acryliques, les polyuréthannes.

14. Composition selon l'une quelconque des revendications précédentes, caractérisée par le fait que le polymère filmogène est présent en une concentration allant de 0,5 % à 40 % en poids, par rapport au poids total de la composition.

15. Composition selon l'une quelconque des revendications précédentes, caractérisée par le fait que le solvant dudit alkyléther est un solvant organique

16. Composition selon l'une quelconque des revendications 1 à 14, caractérisée par le fait que le solvant dudit alkyléther est une huile.

17. Composition selon l'une des revendications précédentes, caractérisée par le fait qu'elle comprend au moins une huile complémentaire non solvante de l'alkyléther de polysaccharide.

18. Composition selon l'une quelconque des revendications précédentes, caractérisée par le fait qu'elle comprend un agent plastifiant du polymère filmogène.

19. Composition selon l'une quelconque des revendications précédentes, caractérisée par le fait qu'elle comprend au moins une silice pyrogénée.

20. Composition selon la revendication 19, caractérisée par le fait que la silice pyrogénée est présente en une quantité allant de 0,1 % à 5 % en poids, de préférence de 0,5 à 1 % en poids, par rapport au poids total de la composition.

21. Composition selon l'une quelconque des revendications précédentes, caractérisée par le fait qu'elle comprend au moins une argile.

22. Composition selon la revendication 21, caractérisée par le fait que l'argile est présente en une teneur allant de 0,1 % à 3 % en poids, par rapport au poids total de la composition, et mieux de 0,5 % à 1,5 % en poids.

23. Composition de soin des ongles ou de vernis à ongles comprenant un polymère filmogène et une phase organique, caractérisé par le fait qu'elle comprend un alkyléther de polysaccharide tel que défini selon les revendications 1 à 10 et en ce que la phase organique comprend au moins un milieu solvant dudit alkyléther.

24. Utilisation d'un alkyléther de polysaccharide formé de motifs comportant au moins deux cycles osidiques différents, chaque motif comportant au moins un groupe hydroxyle substitué par une chaîne alkyle hydrocarbonée saturée comme agent épaississant d'une composition comprenant un polymère filmogène et une phase organique comprenant au moins un milieu solvant dudit alkyléther.

25. Utilisation selon la revendication 24, caractérisée par le fait que l'alkyléther de polysaccharide est un alkyléther d'une gomme choisie parmi la gomme de guar, la gomme de caroube, la gomme de karaya, la gomme adragante et leurs mélanges.

26. Procédé de traitement cosmétique des matières kératiniques et/ou des muqueuses caractérisé par le fait que l'on applique sur les matières kératiniques et/ou sur les muqueuses une composition selon l'une quelconque des revendications 1 à 22.

27. Procédé de maquillage des matières kératiniques et/ou des muqueuses caractérisé par le fait que l'on applique sur les matières kératiniques et/ou sur les muqueuses une composition selon l'une quelconque des revendications 1 à 22.

## Claims

1. Composition comprising a film-forming polymer and an organic phase, characterized in that it comprises a polysaccharide alkyl ether formed of units containing at least two different saccharide rings, each unit containing at least one hydroxyl group substituted with a saturated hydrocarbon-based alkyl chain, the organic phase comprising at least one medium which is a solvent for the polysaccharide alkyl ether.

2. Composition according to Claim 1, characterized in that two to four hydroxyl groups per unit are substituted with a saturated hydrocarbon-based alkyl chain.

3. Composition according to either of the preceding claims, characterized in that the saturated hydrocarbon-based alkyl chain contains from 1 to 24 carbon atoms.

4. Composition according to any one of the preceding claims, characterized in that the saturated hydrocarbon-based alkyl chain contains from 2 to 10 carbon atoms.

5. Composition according to any one of the preceding claims, characterized in that the alkyl chain is chosen from the group formed by the methyl, ethyl, n-propyl, isopropyl, n-butyl, isobutyl and tert-butyl radicals.

6. Composition according to any one of the preceding claims, characterized in that the saccharide rings are chosen from the group formed by mannose, galactose, glucose, furanose, rhamnose and arabinose.

7. Composition according to any one of the preceding claims, characterized in that the polysaccharide alkyl ether is an alkyl ether of a gum chosen from guar gum, carob gum, karaya gum and gum tragacanth, and mixtures thereof.

8. Composition according to any one of the preceding claims, characterized in that the alkyl ether is an alkyl galactomannan with a C₁ to C₆, and better still C₁ to C₃, alkyl chain.

9. Composition according to any one of the preceding claims, characterized in that the polysaccharide alkyl ether is guar gum containing an ethyl chain with a degree of substitution of from 2 to 3.

10. Composition according to any one of the preceding claims, characterized in that the polysaccharide alkyl ether has a weight-average molecular weight of greater than 200,000.

11. Composition according to any one of the preceding claims, characterized in that the polysaccharide alkyl ether is present in an amount such that the ratio (by weight) of the amount of liquid fatty phase to the amount of the said alkyl ether is chosen in the range from 5 to 500.

12. Composition according to any one of the preceding claims, characterized in that the polysaccharide alkyl ether is present in an amount ranging from 0.2 to 20 % of the total weight of the composition, and better still from 1.5 to 8 % of the total weight of the composition.

13. Composition according to any one of the preceding claims, characterized in that the film-forming polymer is chosen from the group formed by nitrocellulose, cellulose acetobutyrate, polyvinyl butyrals, the resins resulting from the condensation of formaldehyde with an arylsulphonamide, alkyd resins, polyesters, acrylics and polyurethanes.

14. Composition according to any one of the preceding claims, characterized in that the film-forming polymer is present in a concentration ranging from 0.5 % to 40 % by weight relative to the total weight of the composition.

15. Composition according to any one of the preceding claims, characterized in that the solvent for the said alkyl ether is an organic solvent.

16. Composition according to any one of Claims 1 to 14, characterized in that the solvent for the said alkyl ether is an oil.

17. Composition according to any one of the preceding claims, characterized in that it comprises at least one complementary oil which is not a solvent for the polysaccharide alkyl ether.

18. Composition according to any one of the preceding claims, characterized in that it comprises a plasticizer for the film-forming polymer.

19. Composition according to any one of the preceding claims, characterized in that it comprises at least one fused silica.

20. Composition according to Claim 19, characterized in that the fused silica is present in an amount ranging from 0.1 % to 5 % by weight, preferably from 0.5 to 1 % by weight, relative to the total weight of the composition.

21. Composition according to any one of the preceding claims, characterized in that it comprises at least one clay.

22. Composition according to Claim 21, characterized in that the clay is present in a content ranging from 0.1 % to 3 % by weight, relative to the total weight of the composition, and better still from 0.5 % to 1.5 % by weight.

23. Nail care or nail varnish composition comprising a film-forming polymer and an organic phase, characterized in that it comprises a polysaccharide alkyl ether as defined according to Claims 1 to 10 and in that the organic phase comprises at least one medium which is a solvent for the said alkyl ether.

24. Use of a polysaccharide alkyl ether formed of units containing at least two different saccharide rings, each unit containing at least one hydroxyl group substituted with a saturated hydrocarbon-based alkyl chain, as a thickener for a composition comprising a film-forming polymer and an organic phase comprising at least one medium which is a solvent for the said alkyl ether.

25. Use according to Claim 24, characterized in that the polysaccharide alkyl ether is an alkyl ether of a gum chosen from guar gum, carob gum, karaya gum and gum tragacanth and mixtures thereof.

26. Cosmetic process for treating keratinous material and/or mucous membranes, characterized in that a composition according to any one of Claims 1 to 22 is applied to the keratinous material and/or to the mucous membranes.

27. Process for making up keratinous material and/or mucous membranes, characterized in that a composition according to any one of Claims 1 to 22 is applied to the keratinous material and/or to the mucous membranes.

## Patentansprüche

1. Zusammensetzung, die ein filmbildendes Polymer und eine organische Phase enthält, dadurch gekennzeichnet, daß sie einen Polysaccharidalkylether enthält, der aus Einheiten gebildet ist, die mindestens zwei verschiedene Zuckerringe aufweisen, wobei jede Einheit mindestens eine Hydroxygruppe enthält, die mit einer gesättigten Alkylgruppe substituiert ist, wobei die organische Phase mindestens ein Lösungsmittelmedium für den Polysaccharidalkylether enthält.

2. Zusammensetzung nach Anspruch 1, dadurch gekennzeichnet, daß pro Einheit 2 bis 4 Hydroxygruppen mit einer gesättigten Alkylgruppe substituiert sind.

3. Zusammensetzung nach einem der vorhergehenden Ansprüche, dadurch gekennzeichnet, daß die gesättigte Alkylgruppe 1 bis 24 Kohlenstoffatome aufweist.

4. Zusammensetzung nach einem der vorhergehenden Ansprüche, dadurch gekennzeichnet, daß die gesättigte Alkylgruppe 2 bis 10 Kohlenstoffatome aufweist.

5. Zusammensetzung nach einem der vorhergehenden Ansprüche, dadurch gekennzeichnet, daß die Alkylgruppe unter Methyl, Ethyl, n-Propyl, Isopropyl, n-Butyl, Isobutyl und tert.-Butyl ausgewählt ist.

6. Zusammensetzung nach einem der vorhergehenden Ansprüche, dadurch gekennzeichnet, daß die Zuckerringe unter Mannose, Galactose, Glucose, Furanose, Rhamnose und Arabinose ausgewählt sind.

7. Zusammensetzung nach einem der vorhergehenden Ansprüche, dadurch gekennzeichnet, daß der Polysaccharidalkylether ein Alkylether eines Gummis ist, das unter Guargummi, Johannesbrotkernmehl, Karaya-Gummi und Tragant und deren Gemischen ausgewählt ist.

8. Zusammensetzung nach einem der vorhergehenden Ansprüche, dadurch gekennzeichnet, daß es sich bei dem Alkylether um ein alkyliertes Galactomannan mit einer C₁₋₆-Alkylgruppe und besser noch einer C₁₋₃-Alkylgruppe handelt.

9. Zusammensetzung nach einem der vorhergehenden Ansprüche, dadurch gekennzeichnet, daß der Polysaccharidalkylether ein Guargummi mit Ethylgruppe ist, das einen Substitutionsgrad von 2 bis 3 aufweist.

10. Zusammensetzung nach einem der vorhergehenden Ansprüche, dadurch gekennzeichnet, daß der Polysaccharidalkylether ein Gewichtsmittel des Molekulargewichts über 200 000 aufweist.

11. Zusammensetzung nach einem der vorhergehenden Ansprüche, dadurch gekennzeichnet, daß der Polysaccharidalkylether in einem solchen Mengenanteil vorliegt, daß das Verhältnis (auf das Gewicht bezogen) der Menge der flüssigen Fettphase und der Menge des Alkylethers im Bereich von 5 bis 500 liegt.

12. Zusammensetzung nach einem der vorhergehenden Ansprüche, dadurch gekennzeichnet, daß der Polysaccharidalkylether in einem Mengenanteil von 0,2 bis 20 % des Gesamtgewichts der Zusammensetzung und besser noch 1,5 bis 8 % des Gesamtgewichts der Zusammensetzung vorliegt.

13. Zusammensetzung nach einem der vorhergehenden Ansprüche, dadurch gekennzeichnet, daß das filmbildende Polymer unter Nitrocellulose, Celluloseacetobutyrat, Butyralpolyvinylverbindungen, Harzen, die bei der Kondensation von Formaldehyd mit einem Arylsulfonamid entstehen, Alkydharzen, Polyestern, Acrylverbindungen und Polyurethanen ausgewählt ist.

14. Zusammensetzung nach einem der vorhergehenden Ansprüche, dadurch gekennzeichnet, daß das filmbildende Polymer in einer Konzentration von 0,5 bis 40 Gew.-%, bezogen auf das Gesamtgewicht der Zusammensetzung, vorliegt.

15. Zusammensetzung nach einem der vorhergehenden Ansprüche, dadurch gekennzeichnet, daß das Lösungsmittel für den Alkylether ein organisches Lösungsmittel ist.

16. Zusammensetzung nach einem der Ansprüche 1 bis 14, dadurch gekennzeichnet, daß das Lösungsmittel für den Alkylether ein Öl ist.

17. Zusammensetzung nach einem der vorhergehenden Ansprüche, dadurch gekennzeichnet, daß sie mindestens ein zusätzliches Öl enthält, das kein Lösungsmittel für den Polysaccharidalkylether darstellt.

18. Zusammensetzung nach einem der vorhergehenden Ansprüche, dadurch gekennzeichnet, daß sie einen Weichmacher für das filmbildende Polymer enthält.

19. Zusammensetzung nach einem der vorhergehenden Ansprüche, dadurch gekennzeichnet, daß sie mindestens eine pyrogene Kieselsäure enthält.

20. Zusammensetzung nach Anspruch 19, dadurch gekennzeichnet, daß die pyrogene Kieselsäure in einem Mengenanteil von 0,1 bis 5 Gew.-% und vorzugsweise 0,5 bis 1 Gew.-%, bezogen auf das Gesamtgewicht der Zusammensetzung, vorliegt.

21. Zusammensetzung nach einem der vorhergehenden Ansprüche, dadurch gekennzeichnet, daß sie mindestens einen Ton enthält.

22. Zusammensetzung nach Anspruch 21, dadurch gekennzeichnet, daß der Ton in einem Mengenanteil von 0,1 bis 3 Gew.-%, bezogen auf das Gesamtgewicht der Zusammensetzung, besser noch 0,5 bis 1,5 Gew.-% vorliegt.

23. Zusammensetzung zur Pflege der Nägel oder Nagellack, die ein filmbildendes Polymer und eine organische Phase enthalten, dadurch gekennzeichnet, daß sie einen Polysaccharidalkylether nach einem der Ansprüche 1 bis 10 enthalten und dadurch, daß die organische Phase mindestens ein Lösungsmittelmedium für den Alkylether aufweist.

24. Verwendung eines Polysaccharidalkylethers, der aus Einheiten gebildet ist, die mindestens zwei verschiedene Zuckerringe aufweisen, wobei jede Einheit mindestens eine Hydroxygruppe enthält, die mit einer gesättigten Alkylgruppe substituiert ist, als Verdickungsmittel in einer Zusammensetzung, die ein filmbildendes Polymer und eine organische Phase enthält, welche mindestens ein Lösungsmittelmedium für den Alkylether aufweist.

25. Verwendung nach Anspruch 24, dadurch gekennzeichnet, daß der Polysaccharidalkylether ein Alkylether eines Gummis ist, das unter Guargummi, Johannesbrotkernmehl, Karaya-Gummi, Tragant oder deren Gemischen ausgewählt ist.

26. Verfahren zur kosmetischen Behandlung von Keratinsubstanzen und/oder Schleimhäuten, dadurch gekennzeichnet, daß auf die Keratinsubstanzen und/oder die Schleimhäute einer Zusammensetzung nach einem der Ansprüche 1 bis 22 aufgebracht wird.

27. Verfahren zum Schminken von Keratinsubstanzen und/oder Schleimhäuten, dadurch gekennzeichnet, daß auf die Keratinsubstanzen und/oder die Schleimhäute eine Zusammensetzung nach einem der Ansprüche 1 bis 22 aufgebracht wird.
